# EUROPEAN PATENT SPECIFICATION

(11) **EP 2 950 825 B1**
(45) Date of publication and mention of the grant of the patent: **11.09.2019**
(21) Application number: 14709772.9
(22) Date of filing: 31.01.2014
(51) Int. Cl.: A61K 47/68, A61K 48/00, A61K 47/64, C12N 15/87, C12Q 1/68

(54) **FUNCTIONALIZED DNA DENDRIMERS FOR GENE DELIVERY TO CELLS**
DNA FUNKTIONALISIERTE DENDRIMERE FÜR GENTRANSPORT IN ZELLEN
COMPLEXES ADN-DENDRIMÈRES FONCTIONNALISÉS UTILISABLES EN VUE DE LA DÉLIVRANCE DE GÈNES EN DIRECTION DE CELLULES

(30) Priority: 01.02.2013 US 201361759558 P
(43) Date of publication of application: 09.12.2015
(73) Proprietor: Genisphere, LLC, Hatfield, PA 19440 (US); Lankenau Institute of Medical Research, Wynnewood, Pennsylvania 19096 (US)
(72) Inventor: GETTS, Robert C., Collegeville Pennsylvania 19426 (US); SAWICKI, Janet, Newtown Square Pennsylvania 19073 (US)
(74) Representative: FRKelly
(86) International application number: PCT/US2014/014104
(87) International publication number: WO 2014/121050

(56) References cited:
- WO-A1-2008/147526
- WO-A1-2011/106481
- WO-A2-01/87350
- WO-A2-2010/017544
- BRAND K ET AL: "TUMOR CELL-SPECIFIC TRANSGENE EXPRESSION PREVENTS LIVER TOXICITY OF THE ADENO-HSVTK&GCV APPROACH", GENE THE, NATURE PUBLISHING GROUP, GB, vol. 5, no. 10, 1 October 1998 (1998-10-01), pages 1363-1371, XP008014785, ISSN: 0969-7128, DOI: 10.1038/SJ.GT.3300728
- Judith C. Keen: "A step towards a new targeted nanotherapy for pancreatic cancer", Cancer biology & therapy, vol. 7, no. 10, 1 October 2008 (2008-10-01), pages 1591-1592, XP055483288, US ISSN: 1538-4047, DOI: 10.4161/cbt.7.10.6758
- Sl Showalter ET AL: "Nanoparticulate delivery of diphtheria toxin DNA effectively kills mesothelin expressing pancreatic cancer cells", Cancer Biology & Therapy, 1 October 2008 (2008-10-01), pages 1584-1590, XP055483295, United States DOI: 10.4161/cbt.7.10.6562 Retrieved from the Internet: URL:https://www.ncbi.nlm.nih.gov/pmc/artic les/PMC3218426/pdf/nihms334791.pdf
- PAPADAKIS E D ET AL: "Promoters and control elements: designing expression cassettes for gene therapy", CURRENT GENE THE, BENTHAM SCIENCE PUBLISHERS LTD, NL, vol. 4, no. 1, 1 March 2004 (2004-03-01), pages 89-113, XP009159935, ISSN: 1566-5232, DOI: 10.2174/1566523044578077
- Weidan Peng ET AL: "Epithelial cell-targeted transgene expression enables isolation of cyan fluorescent protein (CFP)-expressing prostate stem/progenitor cells", TRANSGENIC RESEARCH, vol. 20, no. 5, 11 January 2011 (2011-01-11), pages 1073-1086, XP055483317, NL ISSN: 0962-8819, DOI: 10.1007/s11248-010-9478-2

## Description

### TECHNICAL FIELD

The present invention relates to the field of functionalized DNA dendrimers for delivery of genes to cells, and expression of the genes in the cells.

### BACKGROUND

Gene therapy is a promising treatment for a variety of cellular abnormalities, diseases and conditions because delivery of therapeutic DNA allows for direct treatment of the affected cell. In theory, the ability to target delivery of the DNA to the desired cell, and to control expression once the DNA is taken up by the cell, should provide an improved level of therapeutic efficiency and specificity that is difficult to achieve using conventional therapies. However, in practice, the potential of DNA therapies has not been fully realized. One of the challenges has been the choice of an appropriate vector for delivery of DNA to cells. Viral vectors have been associated with serious collateral effects. As a result, nonviral vectors have been sought. Cationic liposomes have been investigated for gene transfer into cells but have not provided satisfactory results.

More recently, nanoparticles formed by complexing the encoding DNA with a cationic polymer have shown promise for gene therapy, particularly when functionalities are added to improve delivery and therapeutic efficacy (e.g., targeting moieties to improve cell specificity and accumulation, and membrane-permeation moieties to improve cell uptake, and features to prevent degradation). Many of these functionalities are considered necessary for delivery of therapeutic biologics to cells, including genes. Unfortunately, they also increase the complexity of the gene therapeutic and the cost of producing it. In addition, molecular weight, size, charge after DNA condensation and choice of end group termination can have large and unpredictable effects on the transfection potential of the nanoparticle. Further, cationic polymer nanoparticles often lose their DNA delivery capabilities upon conjugation to targeting moieties that would otherwise improve their specificity. However, one of the advantages of cationic polymeric nanoparticles for delivery of DNA to cells is their capacity to deliver large DNA payloads.

DNA dendrimers are complex branched molecules built from interconnected natural or synthetic nucleic acid monomer subunits. Each monomer is composed of two DNA strands that share a region of sequence complementarity located in the central portion of each strand. The two strands anneal to form the monomer, resulting in a structure that has a central double-stranded "waist" bordered by four single-stranded "arms". The single-stranded "arms" at the ends of different monomer types are designed to base-pair with the "arms" of complementary monomer types. This allows directed assembly of the dendrimer as a step-wise series of monomer layers, beginning with a single initiator monomer. It is a useful feature of dendrimers that addition of the last layer of monomers leaves single-stranded "arms" unpaired on the surface of the molecule. The number of surface layer single-stranded "arms" increases with the number of layers. The single-stranded "arms" on the surface are available for attaching a variety of molecules to provide functionality to the dendrimer, including a label or a molecule specific to a particular application. The functional molecules may be conjugated directly to the dendrimer arm, or attached via hybridization to the dendrimer arm. Because of the multiplicity of available single-stranded "arms," multiple different functionalities may be attached to the dendrimer, for example a targeting moiety and a label. Dendrimer have been recognized as providing enhanced detection specificity and sensitivity in a variety of assays.

Targeted expression of the diphtheria toxin (DT) gene in specific cells results in death of those cells. Diphtheria toxin is one of the toxins of choice for cell ablation because its mechanism of action is well-known, and the gene has been cloned, sequenced and adapted for expression in mammalian cells. The precursor polypeptide secreted by *Corynebacterium diphtheria* is subsequently enzymatically cleaved into the A chain and the B chain fragments. The B chain binds to the surface of eukaryotic cells and delivers the A chain into the cytoplasm. DT-A inhibits protein synthesis inside the cell and is extremely toxic; a single molecule is sufficient to kill the cell. The coding sequences for the DT-A subunit have been cloned independently of the DT-B subunit under the control of cell-specific, cis-acting transcriptional regulatory elements to drive expression. After the DT-A subunit kills the cell, it may be released from the dead cells; however, DT-A is not capable of entering neighboring cells.

Thus, there is a need for compositions and methods for targeted delivery of expressible genes to cells which are predictable, specific and provide substantial efficiency in the number of cells that internalize the genes. The present invention addresses these needs.

The prior art includes Papadakis et al, Current Gene Therapy, vol. 4, 2004, pages 89-113, and Weidan Peng et al,,Transgenic Research, vol. 20, 2011 pages 1073-1086. The former discloses promoters for attaining expression in prostate cancers, and the latter discloses K5 promoter mediated gene expression in prostate epithelial basal and luminal cells.

### SUMMARY

The invention to which this specification pertains is set out in the claims appended to this description.

In a first aspect, the invention relates to compositions comprising a DNA dendrimer having one or more DNA sequences linked thereto, the DNA sequences comprising DNA sequences encoding a cytotoxin linked to one or more promoters that regulate expression of the DNA sequences encoding a cytotoxin to produce an RNA coding for the polypeptide. Such DNA dendrimers are referred to herein as "functionalized" DNA dendrimers.

The one or more promoters is substantially transcriptionally active only in a prostate cancer cell, wherein the one or more promoters is the cytokeratin 5 (K5) promoter. This approach may be referred to herein as "targeted gene expression." The prostate cancer-associated promoter is K5.

The DNA sequences encode a cytotoxin. The cytotoxin is Diphtheria toxin A.

DNA sequences may encode an immunomodulatory protein. In specific examples, the immunomodulatory protein is a cytokine or a chemokine. Non-limiting examples of cytokines and chemokines include IL-2, IL-6 and RANTES.

DNA sequences may encode a fluorescent protein. In specific examples, the fluorescent protein is green fluorescent protein (GFP) or cyan fluorescent protein (CFP).

DNA sequences may encode a regulatory RNA. In specific examples, the regulatory RNA is shRNA, miRNA or pre-miRNA, or siRNA. In a further specific example, the regulatory RNA is a shRNA to indolamine 2,3-dioxygenase (IDO).

DNA sequences may encode a plurality of polypeptides or a plurality of regulatory RNAs. The DNA sequences may encode a plurality of polypeptides, a plurality of regulatory RNAs, or a combination of polypeptides and regulatory RNAs.

The DNA dendrimer further comprises a cellular internalization moiety linked thereto. In the disclosure made, the cellular internalization moiety is an antibody that binds to a cell surface protein, a peptide that binds to a cell surface protein, or a ligand that binds to a transferrin receptor. In the invention, the cellular internalization moiety is an antibody or peptide that binds to the transferrin receptor.

In a further aspect, the invention provides pharmaceutical compositions comprising the DNA dendrimer having one or more DNA sequences linked thereto, the DNA sequences comprising DNA sequences encoding a cytotoxin linked to one or more promoters which regulate expression of the DNA sequences encoding a cytotoxin, and a pharmaceutically acceptable carrier. The DNA dendrimer of the pharmaceutical composition of the invention is in its broadest sense the DNA dendrimer as set out in claim 1. The DNA sequences encoding a encode a cytotoxin, wherein the cytotoxin is Diphtheria toxin A.

In specific embodiments of the pharmaceutical compositions, the pharmaceutically acceptable carrier for the functionalized DNA dendrimer is a material that targets the composition for local delivery to cells by facilitating direct exposure of the cells to the functionalized DNA dendrimer. For example, a topical delivery vehicle may be used as a carrier for the functionalized DNA dendrimer and applied directly to the desired location to facilitate uptake of the DNA dendrimer by the cells. In a specific embodiment, the topical delivery vehicle may be a composite material, including a mucoadhesive composite material (e.g., a dextran aldehyde polymer mucoadhesive).

In a further aspect, the functionalized DNA dendrimers according to any of the foregoing embodiments may be used in methods for treating prostate cancer cells. In specific embodiments, the functionalized DNA dendrimer is administered to a patient in need thereof in an amount effective to treat the disease or condition. The DNA dendrimer is targeted to the cells by a cellular internalization moiety. In addition, the DNA dendrimer may be targeted to the cells by the carrier of the pharmaceutical composition as described above. The DNA sequences encoding a cytotoxin will generally be selected for toxicity to the desired cell. The K5 promoter sequences which regulate expression of the DNA sequences encoding the cytotoxin will generally be selected to be substantially transcriptionally active only in the desired cell; however, if a regulatory sequence is deemed not sufficiently transcriptionally specific for the desired cell type, selection of the pharmaceutically acceptable carrier may be used to improve specificity of delivery of the functionalized DNA dendrimer to the desired cell.

In a further disclosure, the functionalized DNA dendrimers described herein may be used in methods for identification and/or visualization of HPV infected epithelial cells, comprising topically applying to the HPV infected epithelial cells a pharmaceutical composition, wherein the DNA sequences regulating gene transcription are specifically transcriptionally active in the HPV infected epithelial cells and the encoded polypeptide is detectable in the HPV infected HPV cells. Accordingly, this disclosure also relates to use of the functionalized dendrimers for identification and/or visualization of HPV infected epithelial cells, either *in vivo, ex vivo,* or *in vitro,* wherein the DNA sequences regulating gene transcription are specifically transcriptionally active in the HPV infected epithelial cells and the encoded polypeptide is detectable in the HPV infected cells.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
Fig. 1A is a color illustration of the structure of the functionalized DNA dendrimers. Fig. 1B is a color photograph showing the results of the *in vitro* experiments described in Example 1. Fig. 1C is a color photograph showing the results of the *in vivo* experiments described in Example 1.
Fig. 2 is a series of color photographs showing the results of the *in vivo* experiments described in Example 2.

### DETAILED DESCRIPTION

The phrase "functionalized DNA dendrimer" and related phrases as used herein refers to a DNA dendrimer that has one or more functionally active moieties attached to its surface. Functionally active moieties include, but are not limited to, moieties that target the DNA dendrimer to a particular cell type, moieties that are therapeutically active, moieties that provide detectability to the DNA dendrimer, and moieties that act within a cell to alter a biological or biochemical activity within the cell. Regulatory RNAs are examples of functionally active moieties, and are non-coding RNAs that exert regulatory influences on biological processes in the cell. Such RNAs include miRNAs and siRNAs. Functionalized DNA dendrimers may also be referred to herein as "nanoDNA," "DNA nanoparticles," "dendrimer nanoparticles," and the like.

The terms "toxic", "toxicity" and similar terms, as used herein in connection with delivery of functionalized DNA dendrimers to cells, refers to the ability of a functionally active moiety attached to the dendrimer to kill the cell.

The phrase "substantially transcriptionally active" only in a particular cell type, "transcriptionally specific" for a specific cell type, or "specifically transcriptionally active," and related phrases, as used herein, refer to a gene construct that is expressed preferentially in a particular (target) cell type relative to other (non-target) cell types. While 100% specificity of expression is desired, the expression in non-target cells at a low level is not excluded. In contrast, "non-transcriptionally specific" refers to a gene construct that is expressed substantially equally in all or most cells types.

The terms "treat," "treating" or "treated" and the like, as used herein, refer to reducing or eliminating a disease or condition; reducing or eliminating at least one symptom of a disease or condition, or; preventing a disease, condition or symptom.

The phrase "pharmaceutically acceptable carrier" refers to any suitable vehicle which can be used to formulate DNA dendrimers for administration to a patient to achieve a therapeutic result. The pharmaceutically acceptable carrier itself generally does not provide additional therapeutic effects. The carrier may be adapted for use in intravenous formulations, topical formulations, oral formulations and the like.

The term "promoter" is used herein in its broadest sense, to refer to any regulatory sequence that enhances, induces or regulates transcription of a downstream DNA coding sequence to produce an RNA complementary to the coding sequence (gene expression).

It has now been found that an expressible genetic construct attached to a DNA dendrimer is a useful delivery vehicle for gene therapy which carries large amounts of the gene construct into the cell. It has been found that the DNA dendrimer and the attached gene construct are effectively internalized by the cell upon contact with the cell surface, and that the polypeptide or regulatory RNA of the gene construct (i.e., the gene product) is effectively expressed within the cell. If the gene product is a toxic molecule, its expression kills the cell. DNA dendrimers are further capable of providing enhanced cell specificity for gene construct delivery to cells, by virtue of cell-specific targeting moieties attached to the DNA dendrimer. In addition, use of promoters in the gene construct that are substantially transcriptionally active only in a specific cell type provides an additional level of cell specificity and further improved therapeutic effect.

A first aspect of the invention provides a DNA dendrimer having linked thereto one or more DNA sequences encoding a cytotoxin (i.e., a coding sequence) operably linked to one or more promoters that regulates transcription of the coding sequence to produce an RNA coding for the cytotoxin (e.g., a promoter). The cytotoxin is Diphtheria toxin A chain and the one or more promoters is the K5 promoter. The promoter that regulates transcription of the coding sequence and the operably linked coding sequence together are referred to herein as a "gene construct." The DNA dendrimer has linked thereto a targeting moiety that directs the DNA dendrimer to a prostate cancer cell and causes binding of the DNA dendrimer to the transferrin receptor. This type of DNA dendrimer is adapted for targeted delivery of the gene construct to the prostate cancer cell, where it can be internalized for expression of the cytotoxin (Diphtheria toxin A chain).

A DNA sequence encoding DT-A is operably linked to the one or more promoters. In a further example, a DNA sequence encoding a siRNA or miRNA that silences expression of a selected gene in the cell upon delivery to the cell via the DNA dendrimer may be operably linked to the one or more promoters.

The DNA sequence that regulates transcription of the coding sequence is a K5 promoter. The K5 promoter is expressed in androgen independent prostate cancer cells.

The DNA sequence that encodes the cytotoxin is functionally active in the cell once the DNA dendrimer is internalized. For example, the coding sequence encodes a polypeptide that is toxic to the cell (such as DT-A).

The DNA dendrimer further includes a targeting moiety linked to the single stranded arms. The targeting moiety may be any protein, polypeptide or ligand that binds to the transferrin receptor on the cell selected for delivery of the dendrimer. The targeting moiety improves binding of the dendrimer to the cell surface, and thereby facilitates effective systemic administration of the dendrimer. For example, the targeting moiety may be an antibody, a hormone, a receptor ligand or a toxin. Specific examples of useful targeting moieties include those that bind to cell surface markers that are expressed exclusively or almost exclusively on the surfaces of pathological cells or on the surfaces of cells of a particular class, type, organ or tissue. These targeting moieties may bind to HER-2/neu (certain breast cancer types), asialofetuin receptor (hepatocytes), viral antigens (virus-infected cells) or scavenger receptors (macrophages).

Alternatively, useful targeting moieties include those that bind to cell surface markers that are overexpressed on the surfaces of pathological cells or on the surfaces of cells of a particular class, type, organ or tissue. These targeting moieties bind to the transferrin receptor.

The targeting moiety may be linked to the dendrimer by a covalent or non-covalent linkage. In a non-covalent linkage, the targeting moiety may be linked to an oligonucleotide that is complementary to a sequence on the single-stranded "arms" of dendrimer, so that the targeting moiety is indirectly hybridized to the dendrimer. Exemplary covalent linkages are known in the art, such as the linkages formed by bifunctional crosslinking agents and hetero bifunctional crosslinking agents, or using "click chemistry".

In additional aspects, the invention provides pharmaceutical compositions comprising any of the foregoing DNA dendrimers. These compositions comprise the DNA dendrimer and at least one pharmaceutically acceptable carrier in a formulation appropriate for the desired route of administration. The DNA dendrimer is present in the pharmaceutical composition in an amount sufficient to deliver enough gene construct to the cell to treat the identified disease or condition when the dendrimer is internalized by the target cell. The amount of dendrimer in the composition may range from 0.1 to 25 weight % based on the total weight of the pharmaceutical composition. Examples of pharmaceutically acceptable carriers include water, stabilizers, thickeners, lubricants, waxes, chelating agents, surfactants, diluents, anti-oxidants, binders, preservatives, coloring agents (such as pigments or dyes), emulsifiers or other conventional constituents of a pharmaceutical formulation. The pharmaceutical composition may be formulated for intravenous administration, topical administration, injection, infusion, or oral administration as is known in the art. The pharmaceutical composition is typically prepared by combining all ingredients under suitable conditions using methods known in the art.

In further aspects, the invention provides the DNA dendrimers and the pharmaceutical compositions comprising the DNA dendrimers for treating prostate cancer in a patient in need thereof. In general, targeting of the gene construct to the desired cells may be achieved by a targeting moiety linked to the DNA dendrimer when the targeting moiety binds to a prostate cancer cell, but does not substantially bind to normal cells.

Second, because the promoter of the gene construct is substantially transcriptionally active only in prostate cancer cells, there is the additional option of systemic administration because, even if the DNA dendrimer is internalized by normal cells, the gene product will not be significantly expressed in the healthy cells. This is referred to as "targeted expression." Improved systemic delivery of the pharmaceutical composition can be achieved by including both targeted delivery and targeted expression features, as discussed above, in the DNA dendrimer.

Also disclosed are methods for making the DNA dendrimers discussed above. Methods for assembly of DNA dendrimers from polynucleotide monomers is known in the art and described, for example, in US Patent No. 5,487,973 and T. Nilsen, et al. (J. Theor. Biol. (1997) 187: 273-284), the disclosures of which are incorporated herein by reference in their entirety. Targeting moieties, may be covalently or non-covalently linked to the dendrimer using various suitable methods known in the art. Such methods include, for example, linkage via disulfide bonds, *N*-hydroxysuccinimide (NHS) ester dependent condensation reactions, bifunctional cross-linking, use of polycationic compounds to bridge the moiety to the dendrimer via charge-charge interactions, or direct or indirect hybridization to the dendrimer arms as disclosed in WO2010/017544. Similarly, the gene construct may be covalently or non-covalently linked to the arms of the dendrimer using these methods. Use of capture sequences for linking molecules to dendrimers by hybridization are also generally described by R. Stears, et al. (Physiol. Genomics (2000) 3:93-99). Other references disclosing methods for attaching molecules to dendrimers by hydrogen bonding (hybridization) include Int. Arch. Allergy Immunol. (2012) 157:31-40, BioTechniques (2008) 44:815-818, J. Clin. Microbiol (2005) 43(7):3255-3259, and Small (2009) 5(15):1784-90.

### Example 1: DNA Dendrimer Delivery of a Cytokeratin 5 (K5) Promoter-Regulated Diphtheria Toxin (DT) Suicide Gene

**2-layer DNA Dendrimer (2n 3DNA) Preparation:** DNA dendrimers were manufactured as previously disclosed (*see, e.g.,* patents 5,175,270, 5,484,904, 5,487,973, 6,110,687 and 6,274,723). Briefly, a DNA dendrimer was constructed from DNA monomers, each of which is made from two DNA strands that share a region of sequence complementarily located in the central portion of each strand. The two strands anneal to form the monomer the resulting structure can be described as having a central double-stranded "waist" bordered by four single-stranded "arms". This waist-plus-arms structure comprises the basic 3DNA® monomer. The single-stranded arms at the ends of each of the five monomer types are designed to interact with one another in precise and specific ways. Base-pairing between the arms of complementary monomers allows directed assembly of the dendrimer through sequential addition of monomer layers. Assembly of each layer of the dendrimer includes a cross-linking process where the strands of DNA are covalently bonded to each other, thereby forming a completely covalent molecule impervious to denaturing conditions that otherwise would cause deformation of the dendrimer structure. In addition, 38 base oligonucleotides that serve as complementary capture oligos are ligated to the 5' ends of available dendrimer arms via a simple T4 DNA ligase dependent ligation reaction, as follows:

**Attaching a capture sequence to a DNA dendrimer:** To attach the transferrin receptor (TfR/CD71) targeting peptide THRPPMWSPVWP (SEQ ID NO:1), the capture oligonucleotide was ligated to 10-15% of the dendrimer arms. The oligonucleotide complementary to the capture oligonucleotide was conjugated to the TfR peptide (Bio-Synthesis, www.biosyn.com) or to anti-CD71 antibodies and hybridized in a molar ratio to occupy all of the available capture sequences. Approximately 2-5 peptides or anti-CD71 antibodies were attached per dendrimer molecule as summarized below.

Small (15-100 nucleotides) DNA or RNA capture oligonucleotides (or other biochemical analogs) were covalently attached to the ends of the dendrimer arms via a simple nucleic acid ligation reaction utilizing a bridging oligonucleotide that overlaps adjacent portions of the dendrimer arm and the capture oligonucleotide, thereby bridging the capture oligonucleotide to the end of the dendrimer arm. The bridging oligonucleotide overlapped as least 5 bases of each of the adjacent dendrimer arm and capture oligonucleotide sequences to facilitate the ligation activity of a nucleic acid ligase enzyme (preferably T4 DNA ligase enzyme), with at least 7 bases of overlap of each sequence preferred. The bridging oligonucleotide may also serve as a nucleic acid blocker for its complementary sequences when the dendrimer is used for specific targeting of non-dendrimer nucleic acids or other molecules.

The following components were added to a microfuge tube:
1. 2 layer DNA dendrimer (500 ng/µL) in IX TE buffer 5.4 µL (2680 ng)
2. a(-)LIG-BR7 Bridging oligo (14mer) (50 ng/µL) 2.7 µL (134 ng)
3. 10X Ligase buffer 10.2 µL
4. 10X Ligase buffer 10.2 µL
5. Cap03 capture oligo (38mer) (50 ng/µL) 4.0 µL (200 ng)
6. T4 DNA Ligase (1 U/µL) 10.0 µL (10 units)

The first four reactants were added together, heated to 65°C and cooled to room temperature. The 5th and 6th reactants were then added and incubated for 45 minutes. The ligation reaction was stopped by adding 2.8 µL of 0.5M EDTA solution. Non-ligated oligonucleotide were removed via the use of 100k cutoff centrifugal filters (Millipore Corp.), and during the purification washes, the buffer was changed into 1x sterile PBS, pH7.4. The capture oligonucleotide is linked to a first single-stranded surface arm of the dendrimer.

In order to target the cell surface and initiate internalization, an antibody or a peptide that binds to the TfR was coupled to the DNA dendrimer. Either antibody to transferrin receptor (TfR/CD71) or a TfR targeting peptide THRPPMWSPVWP (SEQ ID NO:1) were covalently attached to an oligonucleotide that was complementary to the capture oligonucleotide which was previously ligated to the dendrimer (see above). Briefly, the capture oligonucleotide complement (cplCap03), 5'-TTCTCGTGTTCCGTTTGT ACTCTAAGGTGGATTTTT-3' (SEQ ID NO:2), was covalently coupled using commercial chemistry to anti-CD71 antibody (Solulink) or TfR targeting peptide (Biosynthesis) by the 3' end and purified by HPLC to remove excess reagents. These conjugates were then hybridized to dendrimer capture oligonucleotides during the final assembly of the reagents (refer to 3DNA preparation section below).

**K5/CFP and K5/XX 3DNA preparation:** 700µg of pK5/CFP (a plasmid containing the promoter of the cytokeratin 5 gene (*Krt5*) operably linked to the coding sequence of cyan fluorescent protein (CFP)) and 350µg of pK5/XX (a plasmid containing the promoter of the cytokeratin 5 gene only (i.e., no coding sequence)) were restricted with KpnI-HF (New England Biolabs) to linearize the plasmid, which was then dephosphorylated with Shrimp Alkaline Phosphatase (Affymetrix) in a final volume of 7mL and 3.5mL respectively for 2 hours at 37°C. This reaction was subsequently stopped by heating to 80°C for 10 min. An aliquot of each was removed for gel analysis to confirm digestion of the plasmids. Next, oligonucleotides complementary to a second single-stranded surface arm of the dendrimer were covalently linked to the linearized plasmids using commercial chemistry as described above. Specifically, the hybridization oligonucleotide complementary to the second single-stranded dendrimer arm, 10X ligation buffer and T4 DNA ligase were added to the linearized plasmid to a final volume of 8.4 mL for K5/CFP and 4.2 mL for K5/XX. The ligation was started in a beaker water bath at room temperature which was then put at 4°C to slow cool and continue overnight. The next morning, an aliquot was removed from each for gel analysis. A Lonza 1.2% agarose gel was run to determine that ligation of the hybridization oligonucleotide to each plasmid was successful. A combination of DNA dendrimer and each ligated plasmid resulted in a shift of the ligated plasmid into the well with the DNA dendrimer, demonstrating successful ligation of the plasmids. The ligated plasmids were then purified using Amicon 100K columns to remove excess hybridization oligos. The final yield of each purified ligated plasmid was determined using the nanodrop.

The labeled oligonucleotides were prepared by attachment of Cy3 to oligonucleotides complementary to a third single-stranded surface arm of the dendrimer, using methods known in the art.

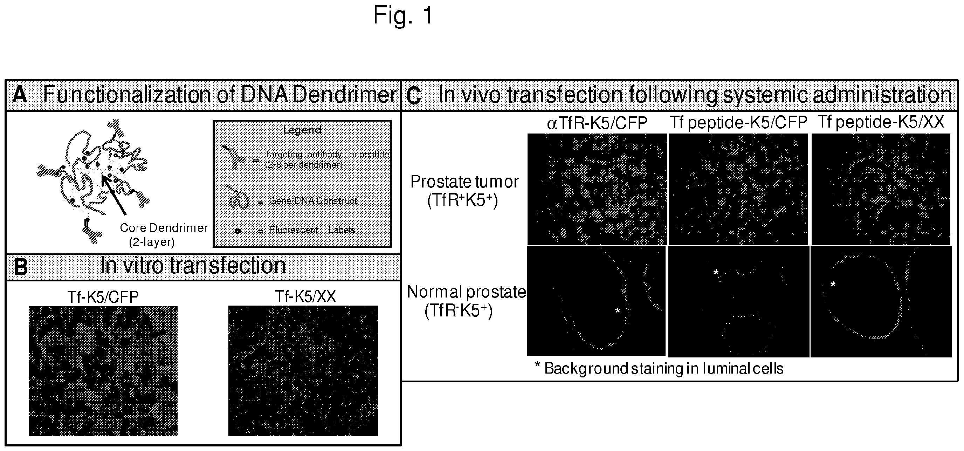

Next, each fully modified 3DNA dendrimer was prepared by hybridizing the TfR peptide/capture complement ("TfR peptide-cplCap03 conjugate") or AntiCD71/capture complement ("AntiCD71-cplCap03 conjugate"), the plasmid ligated to the complement of the second single-stranded dendrimer arm ("ligated plasmid"), and the labeled oligonucleotide complementary to the third single-stranded dendrimer arm ("Cy3 label oligo"), to the dendrimer. This was done by combining the following reagents in the order they are listed. For 2n-Cy3 3DNA-AntiCD71-K5/CFP, 450 µl was prepared to a final concentration of 10 ng/µl 2n 3DNA and 489.8 ng/µl plasmid DNA as follows: 4500 ng 2n (-) 3DNA, 445 ng c(+) Cy3 label oligo, 192.3 ng AntiCD71 -cplCap03 conjugate, 328.9 µl sterile 1X PBS, and 220.4 µg ligated K5/CFP. For 2n-Cy3 3DNA-TfR peptide-K5/CFP, 450 µl was prepared to a final concentration of 10 ng/µl 2n 3DNA and 489.8 ng/µl plasmid DNA as follows: 4500 ng 2n (-) 3DNA, 445 ng c(+) Cy3 label oligo, 192.3 ng TfR peptide-cplCap03 conjugate, 347.7 µl sterile IX PBS, and 220.4ug ligated K5/CFP. For 2n-Cy3 3DNA-TfR peptide-K5/XX, 450 µl was prepared to a final concentration of 10 ng/µl 2n 3DNA and 489.8 ng/µl plasmid DNA as follows: 4500 ng 2n (-) 3DNA, 445 ng c(+) Cy3 label oligo, 192.3 ng TfR peptide-cplCap03 conjugate, 347.7 µl sterile IX PBS, and 220.4 µg ligated K5/XX. Each 3DNA dendrimer was incubated at 37°C for 30 min then stored at 4°C until use. A diagram of the construct is shown in Fig. 1A.

### In vitro and in vivo Prostate Tumor study:

*In vitro:* To confirm *in vitro* binding and delivery, 1 X 10⁵ LAPC4-AS cells (androgen sensitive human prostate cancer cells) were plated in a 24-well plate and cultured at 37°C, 5% CO₂ (balance air) for 2 days. Ten (10) microliters of K5/CFP-TFR (10 ng/ml 3DNA, 500 ng/ml DNA) or K5/XX-TFR dendrimer (10 ng/ml 3DNA, 444 ng/ml DNA) was mixed with 100 microliters OptiMEM medium (Gibco). The medium was removed from the 24-well culture plate, and the dendrimer mixture was added and incubated at 37°C for 4 hrs. One (1) ml of complete medium was added and the culture was continued for 2 days. Cells were immunostained using anti-EGFP antibody (Clonetech 1:1000), and photographed using inverted fluorescent microscope (Fig. 1B).

*In vivo:* For *in vivo* testing of the dendrimer constructs, 5 X 10⁵ PC-3ML/Luc cells (androgen-independent human prostate cancer cells that stably express firefly luciferase) in 100 µl PBS with 20% Matrigel were injected subcutaneously (s.c.) into NOG mice. After 7 weeks, the mice were retro-orbitally injected with 100 µl dendrimer preparation, as prepared above. Twenty-four (24) hours after injection of dendrimer constructs, the mice were sacrificed and tumors, spleen, liver, skin, spleen, kidney, brain, lung and normal prostates were fixed in 10% formalin for 2 hours and embedded in paraffin. Embedded sections were immunostained with anti-GFP antibody (the anti-GFP antibody also recognizes CFP) to determine the level of CFP (Cyan Fluorescent Protein) expression and photographs were taken with a fluorescent microscope. Results are summarized in Fig. 1C. After analyzing the various tissues, it was observed that only the tumor displayed significant expression of CFP, indicating that the dendrimer K5/CFP construct was selectively expressed in tumor cells.

### Example 2: Pancreatic Tumor Targeting (Reference Example)

**2-layer DNA Dendrimer Preparation:** DNA dendrimers were manufactured as previously disclosed (*see, e.g.,* patents 5,175,270, 5,484,904, 5,487,973, 6,110,687 and 6,274,723, each of which is incorporated by reference in its entirety). Briefly, a DNA dendrimer was constructed from DNA monomers, each of which is made from two DNA strands that share a region of sequence complementarily located in the central portion of each strand. The two strands anneal to form the monomer the resulting structure can be described as having a central double-stranded "waist" bordered by four single-stranded "arms". This waist-plus-arms structure comprises the basic 3DNA® monomer. The single-stranded arms at the ends of each of the five monomer types are designed to interact with one another in precise and specific ways. Base-pairing between the arms of complementary monomers allows directed assembly of the dendrimer through sequential addition of monomer layers. Assembly of each layer of the dendrimer includes a cross-linking process where the strands of DNA are covalently bonded to each other, thereby forming a completely covalent molecule impervious to denaturing conditions that otherwise would cause deformation of the dendrimer structure. In addition, 38 base oligonucleotides that serve as complementary capture oligos are ligated to the 5' ends of available dendrimer arms via a simple T4 DNA ligase dependent ligation reaction, as follows:

**Attaching a capture sequence to a DNA dendrimer:** To attach the transferrin receptor (TfR/CD71) targeting peptide THRPPMWSPVWP (SEQ ID NO:1), a capture sequence was ligated to 10-15% of the dendrimer arms. The complementary oligonucleotide to this capture sequence was conjugated to the TfR peptide (Bio-Synthesis, www.biosyn.com) and hybridized in a molar ratio to occupy all of the available capture sequences. Approximately 2-5 peptides were attached per dendrimer molecule as summarized below.

Small (15-100 nucleotides) DNA or RNA capture oligonucleotides (or other biochemical analogs) were covalently attached to the ends of the dendrimer arms via a simple nucleic acid ligation reaction utilizing a bridging oligonucleotide that overlaps adjacent portions of the dendrimer arm and the capture oligonucleotide, thereby bridging the capture oligonucleotide to the end of the dendrimer arm. The bridging oligonucleotide overlapped as least 5 bases of each of the adjacent dendrimer arm and capture oligonucleotide sequences to facilitate the ligation activity of a nucleic acid ligase enzyme (preferably T4 DNA ligase enzyme), with at least 7 bases of overlap of each sequence preferred. The bridging oligonucleotide may also serve as a nucleic acid blocker for its complementary sequences when the dendrimer is used for specific targeting of non-dendrimer nucleic acids or other molecules.

The following components were added to a microfuge tube:
1. 2 layer DNA dendrimer (500 ng/µL) in IX TE buffer 5.4 µL (2680 ng)
2. a(-)LIG-BR7 Bridging oligo (14mer) (50 ng/µL) 2.7 µL (134 ng)
3. 10X Ligase buffer 10.2 µL
4. Nuclease free water 81.7 µL
5. Cap03 capture oligo (38mer) (50 ng/µL) 4.0 µL (200 ng)
6. T4 DNA Ligase (1 U/µL) 10.0 µL (10 units)

The first four reactants were added together, heated to 65°C and cooled to room temperature. The 5th and 6th reactants were then added and incubated for 45 minutes. The ligation reaction was stopped by adding 2.8 µL of 0.5M EDTA solution. Non-ligated oligonucleotide was removed via the use of 100k cutoff centrifugal filters (Millipore Corp.), and during the purification washes, the buffer was changed into 1x sterile PBS, pH7.4. The capture oligonucleotide is linked to a first single-stranded surface arm of the dendrimer.

In order to target the cell surface and initiate internalization, a peptide that binds to the TfR, was coupled to a DNA dendrimer. A targeting peptide THRPPMWSPVWP (SEQ ID NO:1) was covalently attached to an oligonucleotide that is complementary to the capture sequence which was previously ligated to the dendrimer. Briefly, the capture sequence complement (cplCap03), 5'-TTCTCGTGTTCCGTTTGT ACTCTAAGGTGGATTTTT-3' (SEQ ID NO:2), was covalently coupled using commercial chemistry to TfR targeting peptide (Biosynthesis) by the 3' end and purified by HPLC to remove excess reagents. These conjugates were then hybridized to dendrimer capture sequences during the final assembly of the reagents (refer to 3DNA preparation section below).

**MSLN/DT and MSLN/XX 3DNA preparation:** 1 mg of each of two plasmids (MSLN/DT, containing the promoter of the human mesothelin gene (*MSLN*) operably linked to the coding sequence of the A chain of Diphtheria toxin, and; MSLN/XX, containing the MSLN promoter with no coding sequence)was restricted with KpnI- HF (New England Biolabs) to linearize the plasmid, and dephosphorylated with Shrimp Alkaline Phosphatase (Affymetrix) in a final volume of 10 mL for 2 hours at 37°C. This reaction was subsequently stopped by heating to 80°C for 10 min. An aliquot of each was removed for gel analysis to confirm digestion of the plasmid. Next, oligonucleotides complementary to a second single-stranded surface arm of the dendrimer were covalently linked to the linearized plasmids using commercial chemistry as described above. Specifically, the hybridization oligonucleotide complementary to the second single-stranded dendrimer arm, 10X ligation buffer and T4 DNA ligase were added to the linearized plasmid to a final volume of 12 mL. The ligation was started in a beaker water bath at room temperature which was then put at 4°C to slow cool and continue overnight. The next morning, an aliquot was removed from each for gel analysis. A Lonza 1.2% agarose gel was run to determine that ligation of the hybridization oligonucleotide to the plasmid was successful. A combination of DNA dendrimer and each ligated plasmid resulted in a shift of the ligated plasmid into the well with the DNA dendrimer, demonstrating successful ligation of the plasmids. The ligated plasmids were then purified using Qiagen Endo-free plasmid Maxi Kit and Qiagen tip-500. Two tips were used for each plasmid as the maximum capacity of these tips was 500 µg. After elution from the Qiagen tip-500, there was a final step of ethanol precipitation. Pellets were resuspended with sterile IX PBS and the final yield of each purified ligated plasmid was determined using the nanodrop.

The labeled oligonucleotides were prepared by attachment of Cy3 to oligonucleotides complementary to a third single-stranded surface arm of the dendrimer, using methods known in the art.

Next, each fully modified 3DNA reagent was prepared by hybridizing the TfR peptide/capture complement ("TfR peptide-cplCap03 conjugate"), the plasmid ligated to the complement of the second single-stranded dendrimer arm ("ligated plasmid"), and the labeled oligonucleotide complementary to the third single-stranded dendrimer arm ("Cy3 label oligo"), to the dendrimer. This was done by combining the following reagent in the order they are listed. For 2n(-) Cy3 3DNA-TfR peptide-MSLN/DT, 600 µl was prepared to a final concentration of 10 ng/µl 2n 3DNA and 317 ng/µl plasmid DNA as follows : 6000 ng 2n (-) 3DNA, 600 ng c(+) Cy3 label oligo, 256.4 ng TfR peptide-cplCap03 conjugate, 385.2 µl sterile IX PBS, and 190.2 µg ligated MSLN/DT. For 2n(-) Cy3 3DNA-TfR peptide-MSLN/XX, 600 µl was prepared to a final concentration of 10 ng/µl 2n 3DNA and 229 ng/µl plasmid DNA as follows: 6000ng 2n (-) 3DNA, 600 ng c(+) Cy3 label oligo, 256.4 ng TfR peptide-cplCap03 conjugate, 425.2 µl sterile IX PBS, and 137.4 µg ligated MSLN/XX. Each 3DNA dendrimer mixture was then incubated at 37°C for 30 min then stored at 4°C until use.

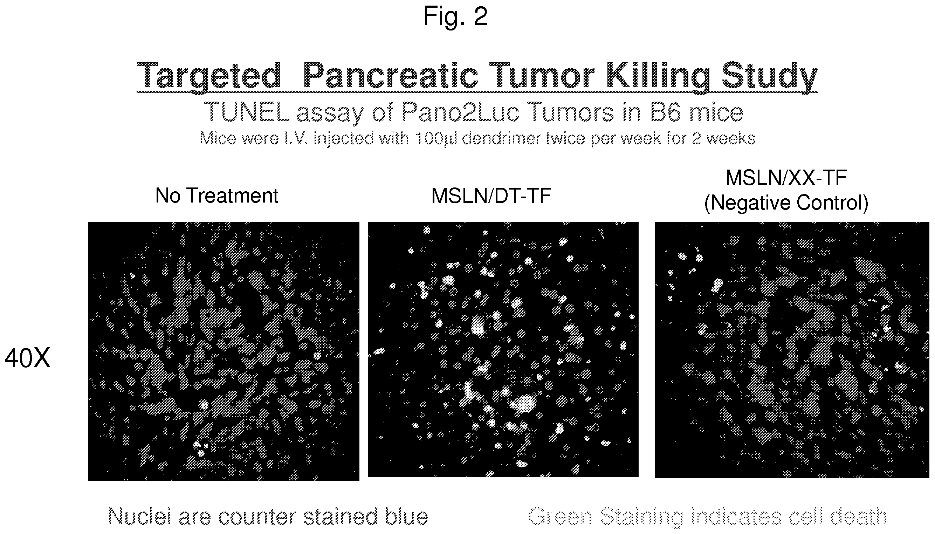

**In Vivo mouse study:** To induce tumor formation in mice 2 X 10⁵ PAN02-Luc cells (mouse pancreatic cancer cells that stably expresses firefly luciferase) in 20 ml Matrigel injected directly into the pancreas of B6 mice. Four weeks later, the mice were retro-orbitally injected with 100 µl of 1x PBS (negative control), MSLN/DT dendrimer (10 ng/ml 3DNA, 311 ng/ml DNA) or with MSLN/XX (10 ng/ml 3DNA, 229 ng/ml DNA). Injections were made two times per week for 2 weeks for a total of 4 doses. The mice were sacrificed 3 days after the last dose, and the tumor, liver, spleen were fixed in 10% formalin for 2 hours, and paraffin-embedded sections were prepared and mounted on slides for further studies. To determine the level of cell death as a result of the treatment, a TUNEL assay was performed using In situ Cell Death Detection Kit (Roche). The results are summarized in Fig. 2 and comparing the conditions, the MSLN/DT dendrimer construct displayed significantly more cell death than either the PBS buffer control or MSLN/XX (no Toxin gene) control.

### Conclusions

The transfection data show that functionalized DNA dendrimers deliver K5/CFP DNA to LAPC-4AS cells *in vitro* and to xenografts following systemic administration. K5/DT kills LAPC-4AI cells *in vitro.* Because the LAPC-4AI cells were derived from LAPC-4AS cells by growing them in androgen-depleted medium (i.e., simulating androgen depletion therapy (ADT)), cell death upon treatment with K5/DT DNA dendrimers is evidence that ADT and nanoDNA-K5/DT will work together. Targeting the TfR enhances binding of TfR peptide-derivatized dendrimer to TfR-expressing LAPC-4 cells, enhances DNA transfection of these cells *in vitro,* and enhances DNA expression in TfR-expressing xenografts upon systemic delivery. Non-TfR tissue is negative for binding.

### Example 3: Targeted DNA Dendrimer-Based Gene Therapy for Cervical Precancerous Lesions (Reference Example)

Anterior gradient protein 2 (AGR2) was recently identified as a biomarker that is expressed at 25-fold higher levels in squamocolumnar (SC) junctional cells of the cervix as compared to neighboring squamous and columnar cells. A previously identified promoter sequence of the *AGR2* gene was identified and shown to have appropriate physiologic activity to target gene expression to SC junctional cells.

**2-layer DNA Dendrimer Preparation:** DNA dendrimers are manufactured as previously disclosed (*see, e.g.,* patents 5,175,270, 5,484,904, 5,487,973, 6,110,687 and 6,274,723, each of which is incorporated by reference in its entirety). Briefly, a DNA dendrimer is constructed from DNA monomers, each of which is made from two DNA strands that share a region of sequence complementarily located in the central portion of each strand. The two strands anneal to form the monomer; the resulting structure can be described as having a central double-stranded "waist" bordered by four single-stranded "arms". This waist-plus-arms structure comprises the basic 3DNA® monomer. The single-stranded arms at the ends of each of the five monomer types are designed to interact with one another in precise and specific ways. Base-pairing between the arms of complementary monomers allows directed assembly of the dendrimer through sequential addition of monomer layers. Assembly of each layer of the dendrimer includes a cross-linking process where the strands of DNA are covalently bonded to each other, thereby forming a completely covalent molecule impervious to denaturing conditions that otherwise would cause deformation of the dendrimer structure. In addition, 38 base oligonucleotides that serve as complementary capture oligos are ligated to the 5' ends of available dendrimer arms via a simple T4 DNA ligase dependent ligation reaction.

**Attaching a capture sequence to a DNA dendrimer:** To attach the transferrin receptor (TfR/CD71) targeting peptide THRPPMWSPVWP (SEQ ID NO:1), a capture sequence is ligated to 10-15% of the dendrimer arms. The complementary oligonucleotide to this capture sequence is conjugated to the TfR peptide (Bio-Synthesis, www.biosyn.com) and hybridized in a molar ratio to occupy all of the available capture sequences. Approximately 2-5 peptides are attached per dendrimer molecule as summarized below.

Small (15-100 nucleotides) DNA or RNA capture oligonucleotides (or other biochemical analogs) are covalently attached to the ends of the dendrimer arms via a simple nucleic acid ligation reaction utilizing a bridging oligonucleotide that overlaps adjacent portions of the dendrimer arm and the capture oligonucleotide, thereby bridging the capture oligonucleotide to the end of the dendrimer arm. The bridging oligonucleotide overlaps at least 5 bases of each of the adjacent dendrimer arm and capture oligonucleotide sequences to facilitate the ligation activity of a nucleic acid ligase enzyme (preferably T4 DNA ligase enzyme), with at least 7 bases of overlap of each sequence preferred. The bridging oligo may also serve as a nucleic acid blocker for its complementary sequences when the dendrimer is used for specific targeting of nondendrimer nucleic acids or other molecules.

The following components are added to a microfuge tube:
1. 2 layer DNA dendrimer (500 ng/µL) in IX TE buffer 5.4 µL (2680 ng)
2. a(-)LIG-BR7 Bridging oligo (14mer) (50 ng/µL) 2.7 µL (134 ng)
3. 10X Ligase buffer 10.2 µL
4. Nuclease free water 81.7 µL
5. Cap03 capture oligo (38mer) (50 ng/µL) 4.0 µL (200 ng)
6. T4 DNA Ligase (1 U/µL) 10.0 µL (10 units)

The first four reactants are added together, heated to 65°C and cooled to room temperature. The 5th and 6th reactants are then added and incubated for 45 minutes. The ligation reaction is stopped by adding 2.8 µL of 0.5M EDTA solution. Non-ligated oligonucleotide is removed via the use of 100k cutoff centrifugal filters (Millipore Corp.), and during the purification washes, the buffer is changed into 1x sterile PBS, pH7.4. The capture oligonucleotide is linked to a first single-stranded surface arm of the dendrimer.

In order to target the cell surface and initiate internalization, an antibody or a peptide that binds to the TfR, is coupled to a DNA dendrimer. Antibody to transferrin receptor (TfR/CD71) or a targeting peptide THRPPMWSPVWP (SEQ ID NO:1) are covalently attached to an oligonucleotide that is complementary to the capture oligonucleotide which was ligated to the dendrimer. Briefly, the capture oligonucleotide complement (cplCAP03), 5'-TTCTCGTGTTCCGTTTGT ACTCTAAGGTGGATTTTT-3' (SEQ ID NO:2), is covalently coupled using commercial chemistry to anti-CD71 antibody (Solulink) or TfR targeting peptide (Biosynthesis) by the 3' end and purified by HPLC to remove excess reagents. These conjugates are then hybridized to dendrimer capture oligonucleotides during the final assembly of the reagents (refer to 3DNA preparation section below).

**AGR2/DT and AGR2/XX 3DNA preparation:** 1 mg of each of the two plasmids (AGR2/DT, containing the promoter of the human anterior gradient 2 (*AGR2*) gene operably linked to the coding sequence of the Diphtheria toxin A chain, and; AGR2/XX, containing the AGR2 promoter with no coding sequence) is restricted with a restriction enzyme to linearize it and dephosphorylated with Shrimp Alkaline Phosphatase (Affymetrix) in a final volume of 10 mL for 2 hours at 37°C. This reaction is subsequently stopped by heating to 80°C for 10 min. An aliquot of each is removed for gel analysis to confirm digestion of the plasmid. Next, oligonucleotides complementary to a second single-stranded surface arm of the dendrimer are covalently linked to the linearized plasmid using commercial chemistry as described above. Specifically, the hybridization oligonucleotide complementary to the second single-stranded dendrimer arm, 10X ligation buffer and T4 DNA ligase are added to the linearized plasmid to a final volume of 12 mL. The ligation is started in a beaker water bath at room temperature which is then put at 4°C to slow cool and continue overnight. The next morning, an aliquot is removed from each for gel analysis. A Lonza 1.2% agarose gel is run to determine that ligation of the hybridization oligonucleotide to the is successful. A combination of DNA dendrimer and each ligated plasmid results in a shift of the ligated plasmid into the well with the DNA Dendrimer, demonstrating successful ligation of the plasmids. The ligated plasmids are then purified using Qiagen Endo-free plasmid Maxi Kit and Qiagen tip-500. Two tips are used for each plasmid as the maximum capacity of these tips is 500 µg. After elution from the Qiagen tip-500, there is a final step of ethanol precipitation. Pellets are resuspended with sterile IX PBS and the final yield of each purified ligated plasmid is determined using the nanodrop.

The labeled oligonucleotides are prepared by attachment of Cy3 to oligonucleotides complementary to a third single-stranded surface arm of the dendrimer, using methods known in the art.

Next, each fully modified 3DNA reagent is prepared by hybridizing the TfR peptide/capture complement ("TfR peptide-cplCap03 conjugate"), the plasmid ligated to the complement of the second single-stranded dendrimer arm ("ligated plasmid"), and the labeled oligonucleotide complementary to the third single-stranded dendrimer arm ("Cy3 label oligo"), to the dendrimer. This was done by combining the following reagent in the order they are listed. For 2n(-) Cy3 3DNA-TfR peptide-AGR2/DT, 600 µl is prepared to a final concentration of 10 ng/µl 2n 3DNA and 317 ng/µl plasmid DNA as follows : 6000 ng 2n (-) 3DNA, 600 ng c(+) Cy3 label oligo, 256.4 ng TfR peptide-cplCap03 conjugate, 385.2 µl sterile IX PBS, and 190.2 µg ligated AGR2/DT. For 2n(-) Cy3 3DNA-TfR peptide-AGR2/XX, 600 µl is prepared to a final concentration of 10 ng/µl 2n 3DNA and 229 ng/µl plasmid DNA as follows: 6000ng 2n (-) 3DNA, 600 ng c(+) Cy3 label oligo, 256.4 ng TfR peptide-cplCao03 conjugate, 425.2 µl sterile IX PBS, and 137.4 µg ligated AGR2/XX. Each 3DNA dendrimer mixture is then incubated at 37°C for 30 min then stored at 4°C until use.

**Ex vivo study:** AGR2/DT and AGR2/XX 3DNA gene expression and cell death is tested following *ex vivo* treatment of human precancerous cervical tissue with a mucoadhesive material combined with the DNA dendrimers. The mucoadhesive material may be a PEG:dextran composite and/or a composite with thermoreversible properties, such as Lutrol F127. Each sample is placed in a well of a 24-well culture dish, overlaid with the adhesive material containing the DNA dendrimers encoding AGR2/DT and AGR2/XX, and incubated for 24 hr. Optical imaging to detect luciferase bioluminescence and TUNEL assay to detect apoptotic cells is performed.
We expect to observe bioluminescence in samples incubated with adhesive material loaded with AGR2/DT DNA dendrimers, but not in control samples treated with AGR2/XX DNA dendrimers. Similarly, we expect to observe many apoptotic cells in samples incubated with AGR2/DT dendrimers, and few apoptotic cells in tissue treated with control dendrimers.

## Claims

1. A composition comprising a DNA dendrimer having linked thereto:
a. one or more DNA sequences encoding a cytotoxin operably linked to one or more promoters, wherein the promoters are substantially transcriptionally active only in prostate cancer cells such that the DNA sequences are expressible substantially only in prostate cancer cells, wherein the cytotoxin is Diphtheria toxin A chain and the one or more promoters is the K5 promoter; and
b. a cellular internalization moiety which targets the DNA dendrimer to the prostate cancer cells; wherein the internalization moiety is an antibody or peptide that binds to the transferrin receptor.

2. The composition of Claim 1, comprising a plurality of linked DNA sequences, wherein the plurality of linked DNA sequences encode a plurality of cytotoxins.

3. A pharmaceutical composition comprising a DNA dendrimer according to any of claims 1-2 and a pharmaceutically acceptable carrier.

4. The pharmaceutical composition of Claim 3, wherein the pharmaceutically acceptable carrier is a mucoadhesive composite.

5. The pharmaceutical composition of Claim 3 or 4, wherein the pharmaceutically acceptable carrier is a carrier for systemic administration.

6. The pharmaceutical composition of Claim 4, wherein the mucoadhesive composite is a dextran aldehyde (PEG:dextran) mucoadhesive composite.

7. The composition of any of Claims 1-2 or the pharmaceutical composition of any of Claims 3-6 for use in treating prostate cancer, wherein the one or more promoters are transcriptionally active in prostate cancer cells and the DNA sequences encoding the cytotoxin is toxic to the prostate cancer cells.

## Patentansprüche

1. Zusammensetzung, die ein DNA-Dendrimer umfasst, das folgende daran gebundene Elemente aufweist:
a. eine oder mehrere DNA-Sequenzen, die ein Zytotoxin kodierten, das funktionell mit einem oder mehreren Promotoren verknüpft ist, wobei die Promotoren im Wesentlichen nur in Prostatakrebszellen transkriptionell aktiv sind, sodass die DNA-Sequenzen im Wesentlichen nur in Prostatakrebszellen exprimiert werden können, wobei das Zytotoxin eine A-Kette eines Diphtherietoxins ist und der eine oder die mehrere Promotoren der K5-Promotor ist; und
b. eine Zellinternalisierungseinheit, die auf das DNA-Dendrimer an den Prostatakrebszellen abzielt; wobei die Internalisierungseinheit ein Antikörper oder ein Peptid ist, das den Transferrin-Rezeptor bindet.

2. Zusammensetzung nach Anspruch 1, die eine Vielzahl von verknüpften DNA-Sequenzen umfasst, wobei die Vielzahl von verknüpften DNA-Sequenzen eine Vielzahl von Zytokinen kodieren.

3. Pharmazeutische Zusammensetzung, die ein DNA-Dendrimer nach einem der Ansprüche 1-2 und einen pharmazeutisch akzeptablen Träger umfasst.

4. Pharmazeutische Zusammensetzung nach Anspruch 3, wobei der pharmazeutisch akzeptable Träger ein mucoadhäsiver Verbundstoff ist.

5. Pharmazeutische Zusammensetzung nach Anspruch 3 oder 4, wobei der pharmazeutisch akzeptable Träger ein Träger zur systemischen Verabreichung ist.

6. Pharmazeutische Zusammensetzung nach Anspruch 4, wobei der mucoadhäsive Verbundstoff ein mucoadhäsiver Dextran-Aldehyd(PEG:dextran)-Verbundstoff ist.

7. Zusammensetzung nach einem der Ansprüche 1-2 oder pharmazeutische Zusammensetzung nach einem der Ansprüche 3-6 zur Verwendung bei der Behandlung von Prostatakrebs, wobei der eine oder die mehreren Promotoren in Prostatakrebszellen transkriptionell aktiv sind und die DNA-Sequenzen das Zytotoxin kodieren, das giftig für die Prostatakrebszellen ist.

## Revendications

1. Composition comprenant un complexe ADN-dendrimère auquel sont liés :
a. une ou plusieurs séquences d'ADN codant pour une cytotoxine liée de manière opérationnelle à un ou plusieurs promoteurs, dans laquelle les promoteurs sont sensiblement et transcriptionnellement actifs uniquement dans les cellules cancéreuses de la prostate, de sorte que les séquences d'ADN peuvent s'exprimer sensiblement uniquement dans des cellules cancéreuses de la prostate, dans laquelle la cytotoxine est la chaîne A de la toxine diphtérique et les un ou plusieurs promoteurs sont le promoteur K5 ; et
b. une fraction d'internalisation cellulaire qui cible le complexe ADN-dendrimère vers les cellules cancéreuses de la prostate ; dans laquelle la fraction d'internalisation est un anticorps ou un peptide qui se lie au récepteur de transferrine.

2. Composition selon la revendication 1, comprenant une pluralité de séquences d'ADN liées, dans laquelle la pluralité de séquences d'ADN liées code pour une pluralité de cytotoxines.

3. Composition pharmaceutique comprenant un complexe ADN-dendrimère selon l'une quelconque des revendications 1 à 2 et un support pharmaceutiquement acceptable.

4. Composition pharmaceutique selon la revendication 3, dans laquelle le support pharmaceutiquement acceptable est un composite muco-adhésif.

5. Composition pharmaceutique selon la revendication 3 ou 4, dans laquelle le support pharmaceutiquement acceptable est un support pour administration systémique.

6. Composition pharmaceutique selon la revendication 4, dans laquelle le composite muco-adhésif est un composite muco-adhésif d'aldéhyde dextrane (PEG : dextrane).

7. Composition selon l'une quelconque des revendications 1 à 2 ou composition pharmaceutique selon l'une quelconque des revendications 3 à 6, destinée à être utilisée dans le traitement du cancer de la prostate, dans laquelle les un ou plusieurs promoteurs sont transcriptionnellement actifs dans les cellules cancéreuses de la prostate et les séquences d'ADN codant pour la cytotoxine sont toxiques pour les cellules cancéreuses de la prostate.
